# EUROPEAN PATENT APPLICATION

(11) **EP 4 332 584 A1**
(43) Date of publication of application: **06.03.2024**
(21) Application number: 22824713.6
(22) Date of filing: 12.05.2022
(51) Int. Cl.: G01N 35/04, G01N 1/10, G01N 21/03

(54) **COMPONENT MEASUREMENT SYSTEM AND COMPONENT MEASUREMENT DEVICE**

(30) Priority: 17.06.2021 JP 2021101064
(71) Applicant: TERUMO Kabushiki Kaisha, Tokyo 151-0072 (JP)
(72) Inventor: HAYASHIDA, Yuma, Nakakoma-gun, Yamanashi 409-3853 (JP); MORIUCHI, Takeyuki, Nakakoma-gun, Yamanashi 409-3853 (JP)
(74) Representative: TBK
(86) International application number: PCT/JP2022/020018
(87) International publication number: WO 2022/264719

(57) **Abstract**

A component measurement system (10) includes an elongated test strip (12) with a thin plate shape having a flow path (20) for accommodating a liquid, and a component measurement device (14) for accommodating the test strip (12) and detecting a component in the liquid. The component measurement device (14) includes a measurement unit that detects a target component in a liquid, and a gripping mechanism (50) that grips the test strip (12) while biasing the test strip (12) from a width direction.

## Description

### Technical Field

The present invention relates to a component measurement system and a component measurement device used for measuring a component contained in a liquid.

### Background Art

A component measurement system (blood glucose meter) that measures the glucose concentration in blood is used for diabetes treatment and determination of a dose of insulin. For example, JP 2006-38857 A discloses a component measurement device using a disposable test strip. The test strip of the component measurement device has a cavity that takes in blood and a reagent that changes in color depending on the concentration of glucose in the blood taken in the cavity. The component measurement device detects a degree of coloration of the reagent in the test strip by a detector of the optical system to detect the glucose concentration in the blood.

The component measurement device disclosed in JP 2006-38857 A has a rail-shaped insertion hole that guides the test strip. This component measurement device measures the glucose concentration while gripping the test strip inserted into the rail-shaped insertion hole from the thickness direction.

### Summary of Invention

However, in the conventional component measurement device, positioning accuracy of the test strip is poor, and a measurement error may occur due to misalignment of the test strip. In addition, in the conventional component measurement device, a measurement portion may be deformed when the test strip is held in the component measurement device.

An object of the present invention is to solve the above problems.

One aspect of the following disclosure is directed to a component measurement system including: a test strip includes: a main body portion with a thin plate shape, a flow path that is formed inside the main body portion, extends from a first end portion toward an opposite second end portion in a longitudinal direction of the main body portion, and accommodates a liquid, and an introduction port provided at the first end; and a component measurement device that accommodates the test strip and detects a component in the liquid, in which the component measurement device includes: a housing having a distal portion and a proximal portion; an insertion hole that is provided inside the housing and accommodates the test strip; a measurement unit that detects a target component in the liquid in at least a part of the flow path in a state in which the test strip is completely accommodated in the insertion hole; and a positioning mechanism that maintains an insertion position of the test strip, the positioning mechanism includes a gripping mechanism that is provided between the insertion hole and the proximal portion of the housing and grips the test strip while biasing the test strip from a width direction.

Another aspect is directed to a component measurement device used in a component measurement system that detects a component in a liquid using a test strip including a main body portion with a thin plate shape, a flow path that is formed inside the main body portion, extends from a first end portion toward an opposite second end portion in a longitudinal direction of the main body portion, and accommodates the liquid, and an introduction port provided at the first end. The component measurement device includes: a housing having a distal portion and a proximal portion; an insertion hole that is provided inside the housing and accommodates the test strip; a measurement unit that detects a component in the liquid in at least a part of the flow path in a state in which the test strip is completely accommodated in the insertion hole; and a positioning mechanism that maintains an insertion position of the test strip, in which the positioning mechanism includes a gripping mechanism that is provided between the insertion hole and the proximal portion of the housing and grips the test strip while biasing the test strip in a width direction.

With the component measurement system and the component measurement device according to the above aspects, measurement errors can be suppressed.

### Brief Description of Drawings

Fig. 1A is a perspective view of a component measurement device of a component measurement system according to an embodiment. Fig. 1B is a perspective view of the component measurement system according to the embodiment. Fig. 1C is a perspective view of a proximal portion side of the component measurement device of Fig. 1A.
Fig. 2 is a perspective view of a test strip of Fig. 1B.
Fig. 3 is an exploded perspective view of the test strip of Fig. 1B.
Fig. 4 is a cross-sectional view of the component measurement device in Fig. 1B.
Fig. 5 is a cross-sectional view (part 1) for explaining an action of the component measurement system in Fig. 1B.
Fig. 6 is a cross-sectional view (part 2) for explaining the action of the component measurement system in Fig. 1B.
Fig. 7 is a cross-sectional view (part 3) for explaining the action of the component measurement system in Fig. 1B.
Fig. 8A is a plan view of a test strip according to a comparative example. Fig. 8B is a plan view of a test strip according to Example 1. Fig. 8C is a plan view of a test strip according to Example 2. Fig. 8D is a plan view of a test strip according to Example 3.
Fig. 9 is an explanatory view illustrating an inclination angle θ of a second inclined portion of an engagement portion of a test strip and a biasing force acting on the test strip by a leaf spring of a gripping mechanism.
Fig. 10 is a graph showing a result obtained by calculating a relationship between the inclination angle θ of the second inclined portion of the test strip and a discharge speed of the test strip.
Fig. 11 is a table showing evaluation results of discharging operations of the test strips according to Examples 1 to 3.

### Description of Embodiments

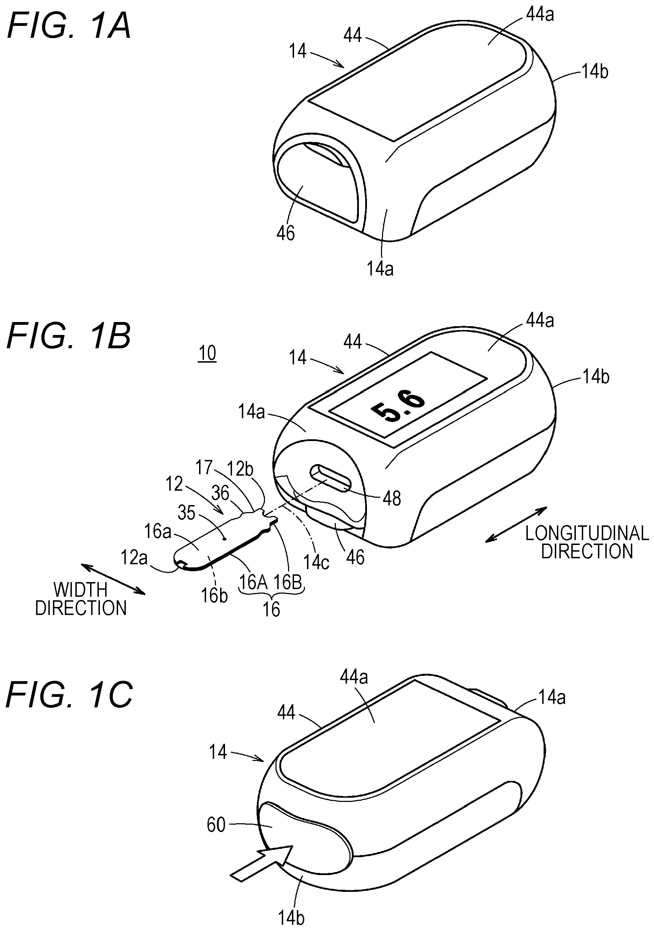

A component measurement system 10 according to the present embodiment illustrated in Figs. 1A to 1C is a component measurement system for personal use operated by a user (patient). The component measurement system 10 is, for example, a blood glucose meter that measures a glucose concentration (blood glucose level) in blood. Note that the component measurement system 10 can also be used by a medical worker to measure a blood glucose level of a patient. The component measurement system 10 includes a test strip 12 and a component measurement device 14. The test strip 12 is a disposable item that is discarded per measurement. The component measurement device 14 measures a blood glucose level with the test strip 12 attached thereto. A direction in which a part of the test strip 12 is exposed from the component measurement device 14 with the test strip 12 attached to the component measurement device 14 is referred to as a distal direction. In an axial direction in which the test strip 12 is inserted, a direction opposite to the distal direction is referred to as a proximal direction.

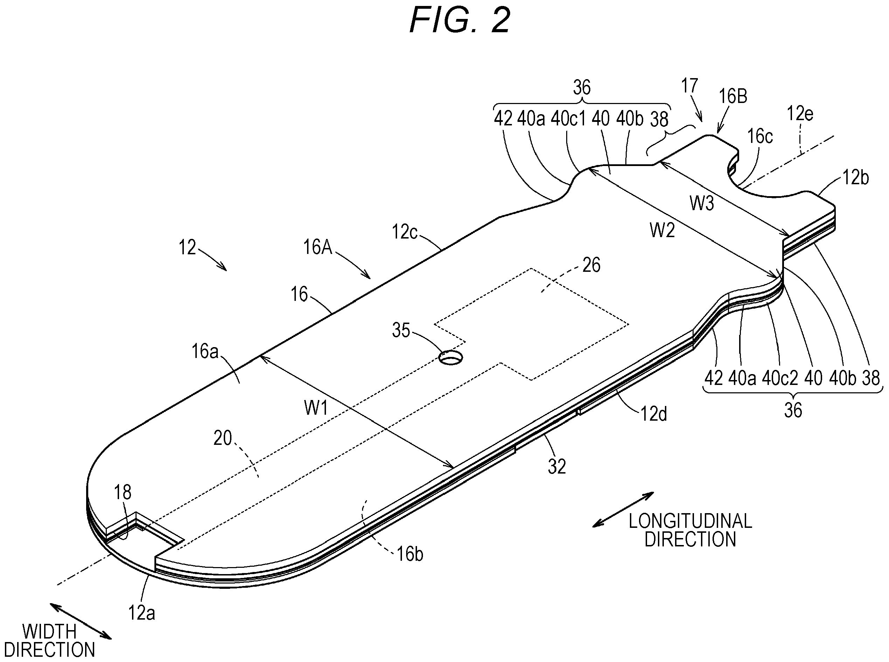

The test strip 12 has a main body portion 16 with an elongated thin plate shape. As illustrated in Fig. 2, one end of the test strip 12 in the direction of the length (longitudinal direction) has a first end portion 12a. The first end portion 12a is formed in a semicircular shape. The other end of the test strip 12 in the longitudinal direction has a second end portion 12b. One end of the test strip 12 in the width direction has a first side portion 12c extending in the longitudinal direction. The other end of the test strip 12 in the width direction has a second side portion 12d extending in the longitudinal direction. The first side portion 12c and the second side portion 12d are formed line-symmetrically with respect to a longitudinal center line 12e, in the direction, of the test strip 12.

The test strip 12 has the second end portion 12b housed in the component measurement device 14. The first end portion 12a of the test strip 12 protrudes to the outside (distal direction side) of the component measurement device 14 with the test strip 12 attached to the component measurement device 14.

An introduction port 18 is formed at the first end portion 12a. A part of a first surface 16a of the main body portion 16 is cut out in a rectangular shape to form the introduction port 18. The introduction port 18 is connected to a flow path 20 described later of the test strip 12. When the user places blood (sample) on the introduction port 18, the blood is introduced into the test strip 12 by capillary force.

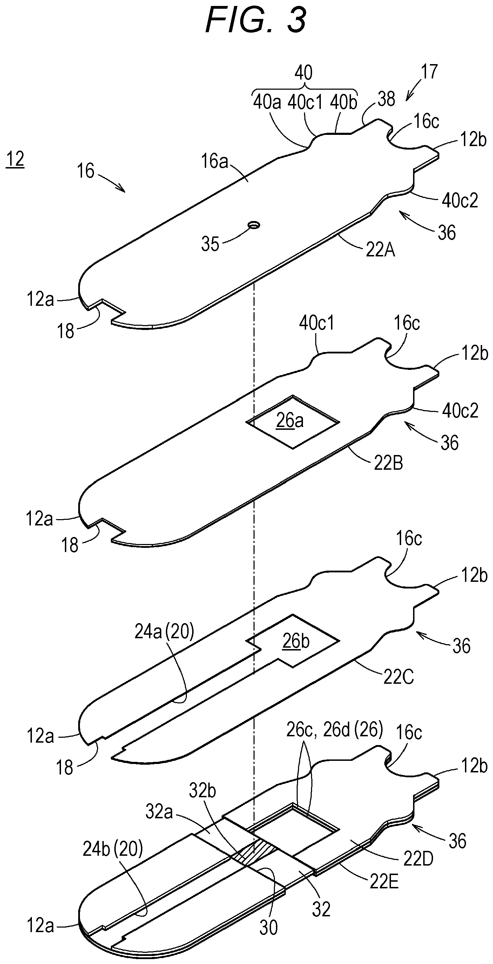

As illustrated in Fig. 3, the main body portion 16 includes a first plate body 22A, a second plate body 22B, a third plate body 22C, a fourth plate body 22D, and a fifth plate body 22E. The first plate body 22A, the second plate body 22B, the third plate body 22C, the fourth plate body 22D, and the fifth plate body 22E are joined in the thickness direction via an adhesive layer such as an adhesive tape. Outer edges of the first plate body 22A to the fifth plate body 22E are formed in substantially the same shape in a plan view as viewed in the thickness direction (except for the introduction port 18). The first end portion 12a of each of the first plate body 22A to the fifth plate body 22E is formed in a semicircular shape. Furthermore, the second end portion 12b of each of the first plate body 22A to the fifth plate body 22E is formed with an arc-shaped notch 16c. In addition to them, the main body portion 16 includes the introduction port 18, the flow path 20, and a buffer space 26.

The first end portion 12a of each of the first plate body 22A to the fourth plate body 22D is formed with the introduction port 18 for taking blood into the main body portion 16. The introduction port 18 is cut out in a rectangular shape in a plan view as viewed from the thickness direction. One end of the flow path 20 communicates with the introduction port 18. The flow path 20 includes a first groove 24a formed in the third plate body 22C and a second groove 24b formed in the fourth plate body 22D. The first groove 24a and the second groove 24b extend linearly along the longitudinal direction of the test strip 12. The dimension in the width direction of each of the first groove 24a and the second groove 24b is narrower than that of the introduction port 18. The introduction port 18 of the fourth plate body 22D and the second groove 24b of the fourth plate body 22D are covered with the fifth plate body 22E. The fifth plate body 22E has a second surface 16b of the main body portion 16. The introduction port 18 and the first groove 24a and the second groove 24b form the flow path 20 by one surface covered with the second plate body 22B and the other surface covered with the fifth plate 22E. The flow path 20 transfers the blood toward the second end portion 12b side by capillary force.

The other end of the flow path 20 communicates with the buffer space 26. The buffer space 26 is a hollow portion having a larger dimension in the width direction than that of the flow path 20. The buffer space 26 includes a first space portion 26a, a second space portion 26b, a third space portion 26c, and a fourth space portion 26d. The first space portion 26a is formed in the second plate body 22B. The second space portion 26b is formed in the third plate body 22C. The third space portion 26c is formed in the fourth plate body 22D. The fourth space portion 26d is formed in the fifth plate body 22E. The buffer space 26 is formed by stacking the first space portion 26a to the fourth space portion 26d. The fourth space portion 26d opens to the second surface 16b of the main body portion 16.

A slit portion 30 extending in the width direction is formed in the fourth plate body 22D and the fifth plate body 22E of the main body portion 16. A reagent plate 32 made of a transparent material is fitted into the slit portion 30, and the reagent plate 32 is attached to the fourth plate body 22D. A first surface 32a of the reagent plate 32 forms a part of the flow path 20. The flow path 20 communicates with the buffer space 26 via the first surface 32a of the reagent plate 32. A reagent 32b is applied onto the first surface 32a of the reagent plate 32. When the reagent plate 32 is attached to the fourth plate body 22D, the reagent 32b is disposed in the flow path 20.

The reagent 32b is a reagent to be dissolved in blood to measure a component in the blood. The reagent 32b is fixed to the reagent plate 32 by being directly applied onto the first surface 32a of the reagent plate 32 and being dried. The reagent 32b causes a color reaction with glucose in blood to generate a color component. As the reagent 32b, for example, a mixed reagent of (i) glucose oxidase (GOD), (ii) peroxidase (POD), (iii) 1(4sulfophenyl)-2,3dimethyl-4amino-5pyrazolone, and (iv) N ethyl-N(2hydroxy-3sulfopropyl)-3,5dimethylaniline sodium salt monohydrate (MAOS) can be used. The reagent 32b may be a mixed reagent of glucose dehydrogenase (GDH) and a tetrazolium salt. Further, the above reagent 32b may contain a pH buffer such as a phosphate buffer, an appropriate mediator, and an additive. The components and types of the reagent 32b are not limited to the above examples.

The component measurement device 14 in Fig. 1A detects a mixture of the reagent 32b and blood. The component measurement device 14 irradiates the reagent plate 32 (reagent 32b) with measurement light having a specific wavelength in a measurement unit. The measurement unit detects the measurement light that has passed through a detection target.

As illustrated in Fig. 2, a window portion 35 through which the measurement light passes is formed in the main body portion 16. As illustrated in Fig. 3, the window portion 35 is a circular hole penetrating the first plate body 22A in the thickness direction. The window portion 35 is formed at a position overlapping the reagent 32b of the reagent plate 32.

Hereinafter, materials of the first plate body 22A to the fifth plate body 22E will be described. The first plate body 22A to the fifth plate body 22E are formed of a resin film using any of a PET resin, a PMMA resin, polyester, polycarbonate, polystyrene, polypropylene, an ABS resin, a COP resin, and a COC resin. Pigments are mixed with these resin films depending on the purpose. The thickness of the resin film used for each of the first plate body 22A to the fifth plate body 22E can be 20 um to 250 um. More preferably, the thickness of the resin film used for each of the first plate body 22A to the fifth plate body 22E can be 20 um to 200 µm.

The first plate body 22A is formed of a black film member having a light shielding property. As the resin film used for the first plate body 22A, a resin material containing carbon black is used. The light shielding rate of the resin film used for the first plate body 22A is preferably 90% or more based on the measurement method of JIS K7605;1976. It is more preferable to use a black film having a light shielding rate of 99% or more for the first plate body 22A. Such a first plate body 22A shields measurement light reaching a portion other than the window portion 35. In addition, the first plate body 22A absorbs stray light of measurement light, which would otherwise affect measurement accuracy.

The second plate body 22B, the third plate body 22C, the fourth plate body 22D, and the fifth plate body 22E are made of transparent resin films. The reagent plate 32 is made of a transparent resin plate.

As shown in Fig. 2, the main body portion 16 of the test strip 12 has a first main body portion 16A on the first end portion 12a side and a second main body portion 16B on the second end portion 12b side. The first main body portion 16A is provided with a configuration necessary for component measurement, such as the flow path 20, the buffer space 26, the introduction port 18, the reagent plate 32, the reagent 32b, and the window portion 35. The second main body portion 16B is provided with a held portion 17 for correctly holding the test strip 12 in the component measurement device 14 during measurement. The held portion 17 has engagement portions 36 and a notch 16c.

The test strip 12 has the notch 16c at the second end portion 12b. In addition, the test strip 12 has a pair of engagement portions 36 on both sides in the vicinity of the second end portion 12b. The notch 16c is formed by cutting out the end surface of the test strip 12 on the second end portion 12b side in an arc shape in a plan view. One end of the test strip 12 in the width direction has a first side portion 12c extending in the longitudinal direction. The other end of the test strip 12 in the width direction has a second side portion 12d extending in the longitudinal direction. The engagement portions 36 are each formed on the first side portion 12c and the second side portion 12d. The engagement portions 36 are a pair of engagement end surfaces provided at the outer edge of the test strip 12 so as to be symmetric with respect to the longitudinal center axis (center line 12e) of the test strip 12 in a plan view.

Each of the engagement portions 36 has a small width portion 38, a convex portion 40, and a concave portion 42 in this order from the second end portion 12b toward the first end portion 12a. The small width portion 38 is formed at a position closest to the second end portion 12b in the engagement portion 36 or at the second end portion 12b. The small width portion 38 constitutes at least a part of the outer edge of the second end portion 12b together with the notch 16c. The small width portion 38 is formed on both outer sides in the width direction of the notch 16c. The small width portion 38 is formed linearly in the longitudinal direction. A width W3 sandwiched between the pair of small width portions 38 in the length of the test strip 12 in the width direction is narrower than a maximum width W1 of the main body portion 16.

The convex portion 40 adjacent to the small width portion 38 is formed between the first end portion 12a and the small width portion 38. The convex portion 40 gently protrudes outward in the width direction in a plan view as viewed in the thickness direction. The convex portion 40 has a first inclined portion 40a, a second inclined portion 40b, and a top portion 40c (40c1, 40c2). The first inclined portion 40a is located at a position adjacent to the top portion 40c between the first end portion 12a and the top portion 40c. The second inclined portion 40b is located at a position adjacent to the top portion 40c between the top portion 40c and the second end portion 12b. The top portion 40c is located between the first inclined portion 40a and the second inclined portion 40b. The first inclined portion 40a has an inclined end surface inclined in a direction of expanding outward in the width direction toward the second end portion 12b. The second inclined portion 40b is formed closer to the second end portion 12b side than the top portion 40c of the convex portion 40. The second inclined portion 40b is an inclined end surface inclined inward in the width direction (that is, the direction in which the width of the test strip 12 is narrowed) as approaching the second end portion 12b. The second inclined portion 40b is inclined at an angle of 30° or more with respect to the center line 12e. More specifically, of angles formed by a straight line obtained by extending the second inclined portion 40b in the direction of the second end portion 12b and the center line 12e, an inclination angle θ formed on the first end portion 12a side is 30° or more and 80° or less. More preferably, the second inclined portion 40b is inclined by 30° to 45° with respect to the center line 12e (that is, 30° ≤ θ ≤ 45°). The convex portion 40 is connected to the small width portion 38 via the second inclined portion 40b. A width W2 between the top portion 40c1 of the convex portion 40 of the first side portion 12c and the top portion 40c2 of the convex portion 40 of the second side portion 12d has the same dimension as the width W1 of the main body portion 16.

The concave portion 42 is located between the first main body portion 16A and the convex portion 40 and is a constriction provided continuously with the first main body portion 16A and the protrusion 40, and has a length in the width direction shorter than the width W1 of the first main body portion 16A and the width W2 of the convex portion 40. The convex portion 40 is connected to the main body portion 16 via the concave portion 42.

As shown in Fig. 1A, the component measurement device 14 has a housing 44. The housing 44 has a distal portion 14a and a proximal portion 14b formed in a semicircular shape that bulges in a plan view from the thickness direction. An upper surface 44a and a lower surface of the housing 44 are formed flatly. The upper surface 44a is provided with a display unit that displays a measurement result of a blood glucose level. The distal portion 14a of the component measurement device 14 is provided with a lid portion 46. The lid portion 46 slides in a direction perpendicular to the extending direction of the upper surface 44a. An insertion hole 48 into which the test strip 12 is inserted is formed in a side surface of the housing 44 covered with the lid portion 46. The insertion hole 48 is exposed by sliding the lid portion 46. The lid portion 46 also serves as a power switch of the component measurement device 14. When the insertion hole 48 is exposed, the component measurement device 14 is activated.

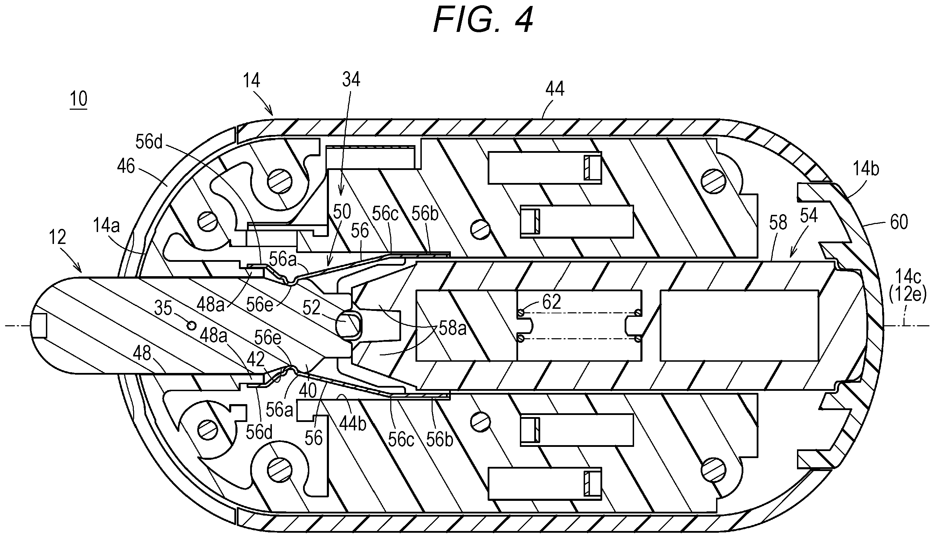

As illustrated in Fig. 4, an insertion hole 48 is provided inside the housing 44 of the component measurement device 14. The insertion hole 48 extends from the distal portion 14a toward the proximal portion 14b of the housing 44. The dimension in the width direction of the inside of the insertion hole 48 is substantially the same as the width W1 (see Fig. 2) of the main body portion 16 of the test strip 12. In addition, the dimension in the thickness direction inside the insertion hole 48 is substantially the same as the dimension in the thickness direction of the test strip 12. Therefore, the insertion hole 48 accommodates the test strip 12 in the width direction and the thickness direction without rattling. The proximal end of the insertion hole 48 is connected to an internal space 44b of the housing 44. The length of the insertion hole 48 in the direction of the center line 14c is shorter than the length of the test strip 12 in the longitudinal direction. When the test strip 12 is inserted into the insertion hole 48, the second end portion 12b of the test strip 12 is accommodated in the internal space 44b via the insertion hole 48.

The housing 44 internally includes a gripping mechanism 50, a receiving portion 52, a discharge mechanism 54, and a measurement unit (not illustrated). The gripping mechanism 50 and the receiving portion 52 are included in a positioning mechanism 34. The gripping mechanism 50 includes a pair of opposing leaf springs 56. The leaf springs 56 are each disposed outside the side portion of the internal space 44b, and face each other with respect to the center line 14c. The pair of leaf springs 56 holds the test strip 12 from both sides in the width direction of the test strip 12. The leaf spring 56 has a distal portion 56a abutting on the test strip 12 and a proximal portion 56b joined to an inner wall of the internal space 44b. In the leaf spring 56, a bent portion 56c is formed between the distal portion 56a and the proximal portion 56b.

The distance between the center line 14c of the internal space 44b and the insertion hole 48 and the distal portion 56a is shorter than the distance between the center line 14c and the proximal portion 56b. The distal portion 56a has a sliding protrusion 56e and a locking portion 56d. The locking portion 56d is formed at the extreme tip of the distal portion 56a. In a state in which the test strip 12 is not inserted into the internal space 44b, the locking portion 56d abuts on the outside in the width direction of the side wall portion of the insertion hole 48. The locking portion 56d prevents the distal portion 56a from approaching the center line 14c. The locking portion 56d abuts on the side wall portion of the insertion hole 48 in a state of being biased toward the center line 14c by the elastic restoring force of the leaf spring 56.

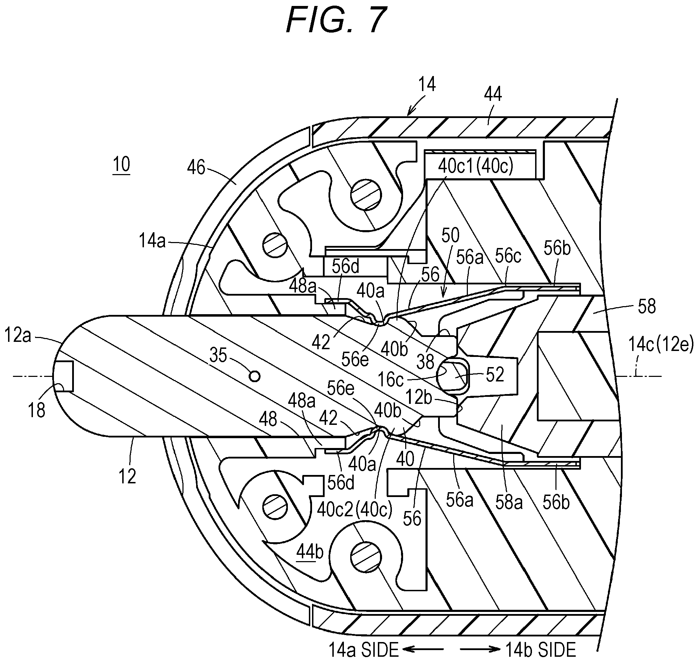

The sliding protrusion 56e is formed on the proximal side of the locking portion 56d. The sliding protrusion 56e has an arc shape protruding inward in the width direction in a plan view from the thickness direction of the test strip 12. When the test strip 12 is inserted into the internal space 44b, the engagement portion 36 slides between the pair of leaf springs 56 while abutting on the sliding protrusion 56e. As illustrated in Fig. 7, the sliding protrusion 56e abuts on the first inclined portion 40a of the convex portion 40 in the completed state in which the test strip 12 is inserted. In this manner, the sliding protrusion 56e biases the test strip 12 toward the proximal portion 14b of the housing 44 by the elastic restoring force of the leaf spring 56 and the inclination direction of the first inclined portion 40a. That is, the sliding protrusion 56e holds the test strip 12 to be drawn into the housing 44. Immediately before the user completes the insertion of the test strip 12, the pair of leaf springs 56 biases the test strip 12 in a direction in which the test strip 12 is drawn into the component measurement device 14, thereby assisting the insertion operation. Thus, the test strip 12 can be reliably guided to a predetermined position.

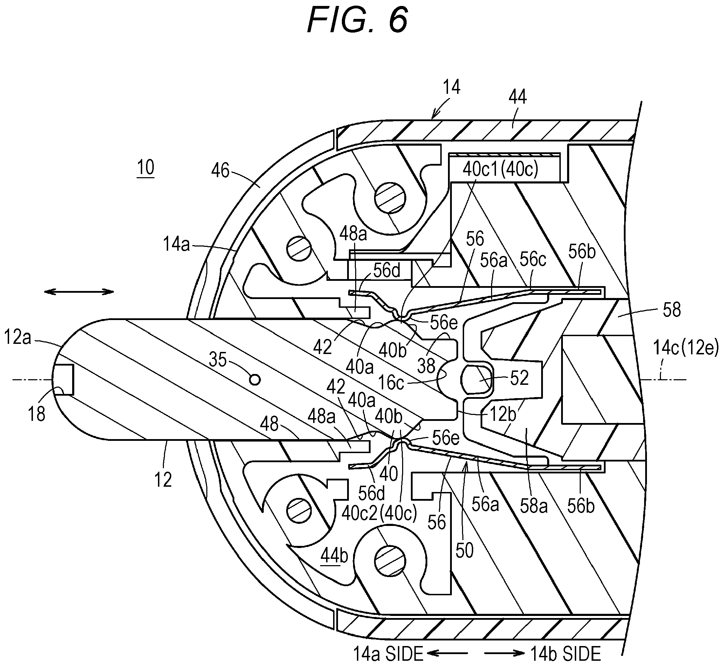

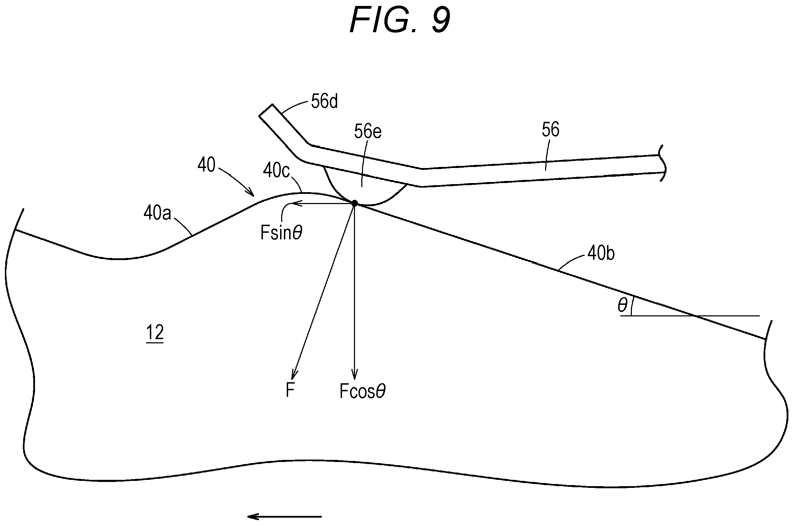

As illustrated in Fig. 6, when the test strip 12 is moved in the discharging direction with respect to the housing 44 during the ejecting operation of discharging the test strip 12, the sliding protrusion 56e gets over the convex portion 40 from the concave portion 42 to transit to a state of abutting on the second inclined portion 40b. In this process, after the sliding protrusion 56e gets over the convex portion 40, the sliding protrusion 56e biases the test strip 12 toward the distal portion 14a of the housing 44 by the elastic restoring force of the leaf spring 56 and the inclination direction of the second inclined portion 40b. A biasing force F of the sliding protrusion 56e (leaf spring 56) acting on the second inclined portion 40b also acts in a direction in which the test strip 12 is discharged toward the distal portion 14a (Fsinθ) (see Fig. 9).

The receiving portion 52 is a columnar member extending in the thickness direction of the housing 44. The receiving portion 52 prevents the test strip 12 from moving in the proximal direction (direction of the proximal portion 14b side of the housing 44). The outer diameter of the receiving portion 52 is sized to fit into the notch 16c. When the test strip 12 is inserted, as illustrated in Fig. 7, the notch 16c of the test strip 12 abuts on the receiving portion 52 in a state of being biased by the biasing force F of the sliding protrusion 56e and the first inclined portion 40a. The receiving portion 52 is fitted to the notch 16c to position the second end portion 12b of the test strip 12 in the width direction and the longitudinal direction.

As illustrated in Fig. 4, the discharge mechanism 54 includes a pressing piece 58 and an eject button 60 for operating the pressing piece 58. The pressing piece 58 extends in the vicinity of the proximal portion 14b along the center line 14c of the housing 44 and the insertion hole 48. The eject button 60 is exposed on the outer surface of the proximal portion 14b of the housing 44. The eject button 60 is operatively connected to the pressing piece 58 inside the housing 44. When the user presses the eject button 60 toward the distal portion 14a, the eject button 60 and the pressing piece 58 are displaced toward the distal portion 14a.

Two pressing portions 58a are provided at the tip of the pressing piece 58. The pressing portion 58a can move toward the insertion hole 48 during the ejection operation to push out the test strip 12 toward the distal portion 14a of the housing 44. A coil spring 62 is attached to the inside of the pressing piece 58 in the longitudinal direction. The coil spring 62 biases the pressing piece 58 and the eject button 60 toward the proximal portion 14b. When the eject button 60 is pressed with a force larger than the resilient force of the coil spring 62, the eject button 60 and the pressing piece 58 are displaced toward the distal portion 14a. In a state in which the eject button 60 is not pressed, the distal end surface of the pressing portion 58a of the pressing piece 58 is located on the proximal side of the receiving portion 52. Therefore, the pressing portion 58a is separated from the test strip 12 except during the ejecting operation.

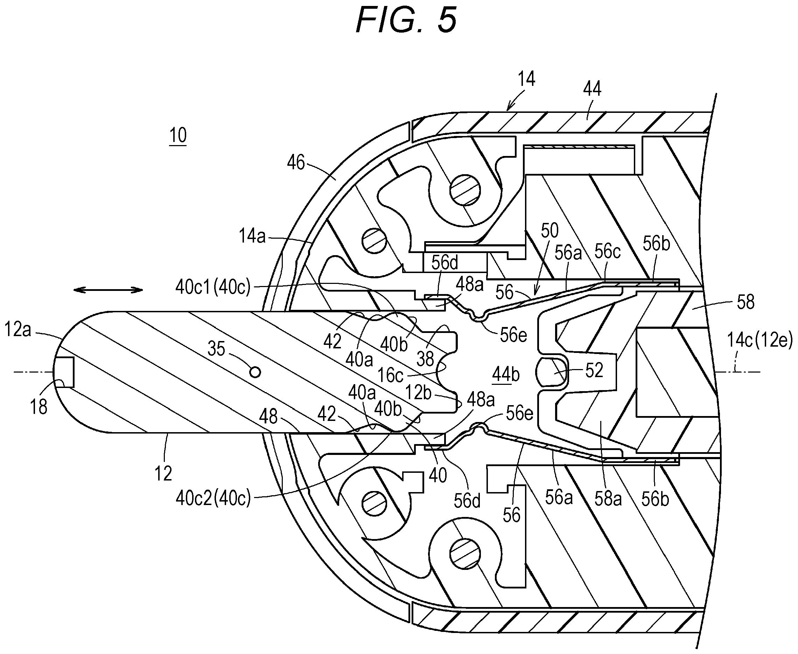

In the eject operation, the pressing portion 58a pushes out the test strip 12 toward the distal portion 14a. As a result, as illustrated in Fig. 6, the sliding protrusion 56e of the leaf spring 56 gets over the convex portion 40 of the test strip 12. The elastic restoring force of the leaf spring 56 accelerates the test strip 12 toward the distal portion 14a while sliding the sliding protrusion 56e on the second inclined portion 40b. As a result, as shown in Fig. 5, the test strip 12 is discharged from the insertion hole 48 at a predetermined speed.

Although not particularly illustrated, the component measurement device 14 includes a measurement unit including an irradiation unit that irradiates a portion corresponding to the window portion 35 of the test strip 12 with measurement light and a light receiving unit. The measurement unit detects a blood glucose level from a detection value of the light receiving unit, and displays the detection result on the upper surface 44a.

The component measurement system 10 will be described.

As illustrated in Fig. 5, the user inserts the test strip 12 into the insertion hole 48 from the second end portion 12b side of the test strip 12. The test strip 12 is inserted into the internal space 44b toward the proximal side. In a state in which at least a part of the convex portion 40 of the engagement portion 36 is along the side wall of the internal space 44b, the locking portion 56d of the leaf spring 56 is in a state of being held to abut on the outer side in the width direction of the proximal end (stopper 48a) of the side wall of the insertion hole 48. At this time, the leaf spring 56 does not abut on the small width portion 38 of the engagement portion 36 of the test strip 12. In addition, in a plan view, the engagement portion 36 of the test strip 12 is inserted between the gripping mechanisms 50 having the pair of leaf springs 56. In this manner, a displaceable range of the leaf spring 56 in the width direction is restricted by the locking portion 56d and the proximal end of the side wall of the insertion hole 48.

As shown in Fig. 6, when the test strip 12 is further inserted, firstly, the second inclined portion 40b of the convex portion 40 of the engagement portion 36 abuts on the sliding protrusion 56e. The convex portion 40 pushes and spreads the leaf spring 56 outward in the width direction. More specifically, a contact position between the sliding protrusion 56e and the engagement portion 36 moves from the second inclined portion 40b toward the top portion 40c as the test strip 12 is inserted. The distance between the pair of sliding protrusions 56e increases from the length between the second inclined portions 40b facing each other in the test strip 12 depending on the length (W2) between the facing top portions 40c (40c1, 40c2). When the test strip 12 is inserted to the proximal side, the sliding protrusion 56e of the leaf spring 56 gets over the top portion 40c, and the sliding protrusion 56e abuts on the first inclined portion 40a of the convex portion 40 (Figs. 4 and 7). By the inclination direction of the first inclined portion 40a and the biasing force F of the sliding protrusion 56e, the test strip 12 is biased so as to be drawn toward the proximal portion 14b. As a result, the notch 16c of the second end portion 12b of the test strip 12 is fitted into the receiving portion 52.

As described above, the component measurement device 14 grips the test strip 12 and positions it at a predetermined position. Since the component measurement device 14 grips the test strip 12 without pressing it in the thickness direction, it is possible to suppress deformation in the thickness direction of the flow path 20 (see Fig. 3) inside the test strip 12. As a result, the component measurement device 14 can prevent the variation in an optical path length of the measurement light of the test strip 12, and can suppress a measurement error.

After the measurement is completed (Fig. 7), the user performs an operation to push out the eject button 60 illustrated in Fig. 4. As a result, the test strip 12 is pushed out toward the distal portion 14a and is separated from the component measurement device 14.

As the eject button 60 is pressed, the pressing piece 58 abuts on the proximal end of the test strip 12 (Fig. 7) to push out the test strip 12 in the discharge direction (the direction of the distal portion 14a side). The test strip 12 is moved in the direction of the distal portion 14a side by the pressing piece 58. As a result, the portion where the sliding protrusion 56e and the engagement portion 36 abut on each other moves from the first inclined portion 40a to the position beyond the top portion 40c. The moving length of the pressing piece 58 in the longitudinal direction necessary for discharging the test strip 12 is equal to or longer than the displacement amount of the sliding protrusion 56e in the longitudinal direction from the first inclined portion 40a to the position beyond the top portion 40c.

Next, as shown in Fig. 6, the position where the sliding protrusion 56e and the engagement portion 36 abut on each other moves to a position where the sliding protrusion gets over the top portion 40c to abut on the second inclined portion 40b. After getting over the top portion 40c, the test strip 12 is biased so as to be pushed out toward the distal portion 14a side by the biasing force F toward the inside in the width direction of the leaf spring 56 and the second inclined portion 40b inclined toward the proximal portion 14b side (that is, the length in the width direction of the test strip 12 becomes narrower toward the proximal portion 14b), and is discharged. While the sliding protrusion 56e is abutting on at least a part of the top portion 40c and the second inclined portion 40b, the locking portion 56d is separated from the side wall portion of the insertion hole 48. The sliding protrusion 56e reaches the second inclined portion 40b, and the biasing force F in the discharge direction is applied to the test strip 12. When the abutment state between the sliding protrusion 56e and the engagement portion 36 of the test strip 12 is released, the sliding protrusion 56e separates from the test strip 12. Thereafter, the leaf spring 56 transitions to a state in which the locking portion 56d abuts on the outer side of the insertion hole 48 in the proximal portion width direction by the restoring force of the leaf spring 56 itself (Fig. 5).

The length between the pair of locking portions 56d facing each other is restricted by the side wall of the proximal portion of the insertion hole 48. In this way, a displacement range of the leaf spring 56 is restricted, and thus, immediately before the test strip 12 is locked to the leaf spring 56 and immediately after the test strip 12 is separated from the holding state by the leaf spring 56, a pair of sliding protrusions 56e facing each other is prevented from coming into contact with the small width portion 38. Therefore, the pair of locking portions 56d facing each other and the side wall end portions on both sides in the width direction of the proximal portion of the insertion hole 48 prevent an occurrence of frictional resistance due to a contact of the sliding protrusion 56e with the small width portion 38 during the insertion and removal of the test strip 12 into and from the component measurement device 14, and enable a smooth ejection operation.

Hereinafter, examples and comparative examples of the test strip 12 will be described. The test strip 12 is formed by laminating a plurality of resin films and punching the laminate with a punching die. Therefore, there is a possibility that an adhesive component having a relatively large frictional resistance is exposed at the engagement portions 36 of the side portions 12c and 12d of the test strip 12. In such a case, even if the biasing force F of the leaf spring 56 is appropriately adjusted, the test strip 12 may not be smoothly discharged from the component measurement device 14. When the biasing force F of the leaf spring 56 is too high, it is difficult to insert the test strip 12. Therefore, the inventors of the present application have studied the shape of the test strip 12.

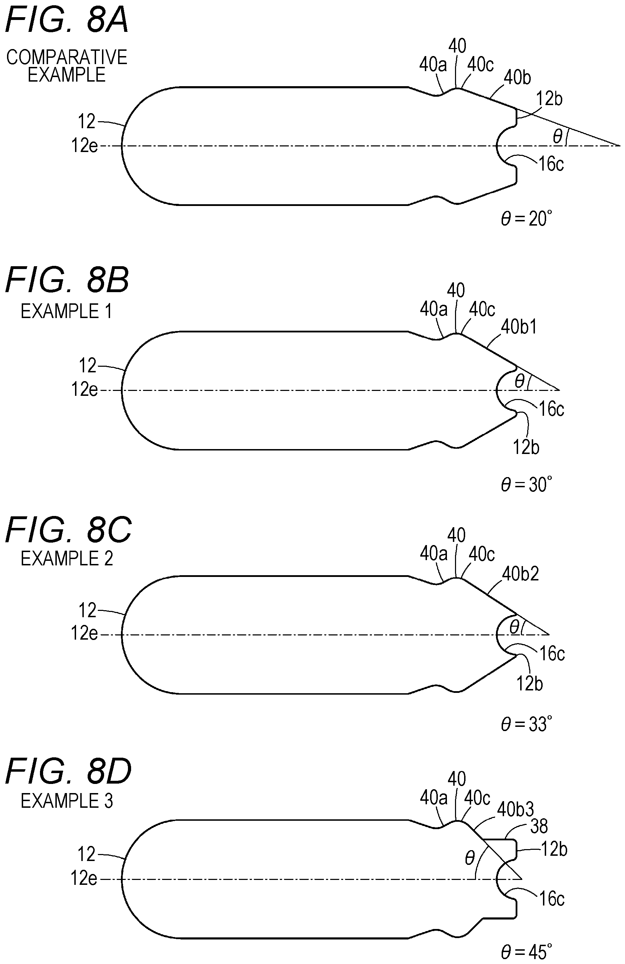

A test strip 12 according to the comparative example shown in Fig. 8A has an engagement portion 36 having a convex portion 40 in the vicinity of a second end portion 12b. The convex portion 40 of the comparative example has a second inclined portion 40b extending linearly to the second end portion 12b of the test strip 12. An angle (an acute angle) between a virtual extension line obtained by extending the second inclined portion 40b of the comparative example toward a proximal side and a center line 12e of the test strip 12 is 20°. That is, in the comparative example, the second inclined portion 40b is inclined outward by 20° in the width direction with respect to the center line 12e toward a distal end side.

A test strip 12 according to Example 1 shown in Fig. 8B has an engagement portion 36 having a convex portion 40 in the vicinity of a second end portion 12b. The convex portion 40 of Example 1 has a second inclined portion 40b1 extending linearly to the second end portion 12b of the test strip 12. An angle (an acute angle) between a virtual extension line obtained by extending the second inclined portion 40b1 of Example 1 toward a proximal side and a center line 12e of the test strip 12 is 30°. That is, in Example 1, the second inclined portion 40b1 is inclined outward by 30° in the width direction with respect to the center line 12e toward a distal end side.

A test strip 12 according to Example 2 shown in Fig. 8C has an engagement portion 36 having a convex portion 40 in the vicinity of a second end portion 12b. The convex portion 40 of Example 2 has a second inclined portion 40b2 extending linearly to the second end portion 12b of the test strip 12. An angle (an acute angle) between a virtual extension line obtained by extending the second inclined portion 40b2 of Example 2 toward a proximal side and a center line 12e of the test strip 12 is 33°. That is, in Example 2, the second inclined portion 40b2 is inclined outward by 33° in the width direction with respect to the center line 12e toward a distal end side.

A test strip 12 according to Example 3 illustrated in Fig. 8D has the same configuration as that described with reference to Figs. 2 to 7. An angle (an acute angle) between a virtual extension line obtained by extending a second inclined portion 40b3 of the test strip 12 toward a proximal side and a center line 12e of the test strip 12 is 45°. That is, in Example 3, the second inclined portion 40b3 is inclined outward by 45° in the width direction with respect to the center line 12e toward a distal end side.

As illustrated in Fig. 9, when a biasing force F of a leaf spring 56 acts on a second inclined portion 40b of a test strip 12, a propulsive force of F × sinθ acts on a second inclined portion 40b having an inclination angle θ toward a distal end side.

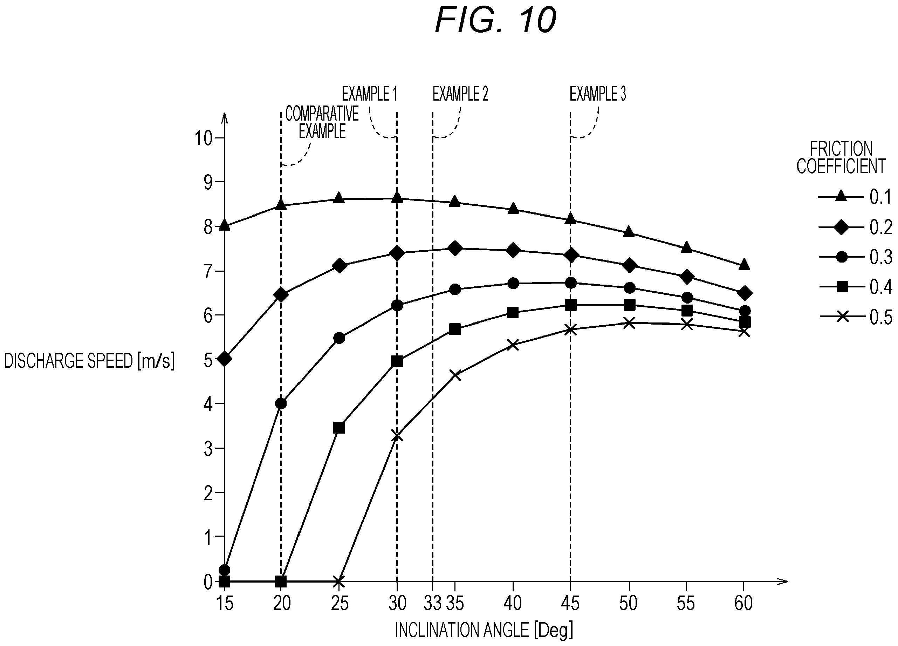

Therefore, as shown in Fig. 10, the inventors obtained the discharge speed of the test strip 12 when the inclination angle θ and the friction coefficient of the second inclined portion 40b of the test strip 12 were changed for the comparative example and each example. A friction coefficient of 0.1 represents a friction coefficient of the ideal second inclined portion 40b. A friction coefficient of 0.5 represents a friction coefficient in a state in which the exposure amount of the adhesive having adhesiveness is large in the second inclined portion 40b.

In the case of a friction coefficient of 0.5, when an inclination angle of the second inclined portion 40b is 25° or less, the discharge speed of the test strip 12 is 0. In this case, an initial speed cannot be applied to the test strip 12, and the test strip 12 fails in discharge. On the other hand, as in Examples 1 to 3, when an inclination angle of the second inclined portion 40b is 30° or more, the test strip 12 can be discharged from the component measurement device 14 at a speed of 3 m/sec or more. That is, when an inclination angle of the second inclined portion 40b is 30°, the test strip 12 is reliably discharged even when the adhesive is exposed to the side portions 12c and 12d of the test strip 12.

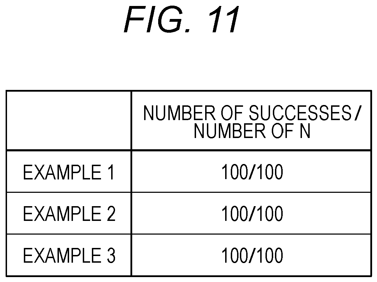

Next, as shown in Fig. 11, the inventors confirmed the discharging operation of the test strip 12 of Examples 1 to 3 using the component measurement device 14. As a determination criterion, when the component measurement device 14 to which the test strip 12 is attached is discharged so that the insertion hole 48 opens downward, it is determined that the discharge succeeds when the second inclined portion 40b of the test strip 12 is discharged at a predetermined speed or more without being caught by the leaf spring 56 or the insertion hole 48. The inventors prepared 100 test strips 12 for each of Examples 1 to 3 and performed an operation test. As shown, the component measurement device 14 successfully ejected all the test strips 12 of Examples 1 to 3. As described above, by setting the inclination angle of the second inclined portion 40b to 30° or more, the test strip 12 can be reliably discharged. As a result, the user can discard the test strip 12 without touching it after use to which blood is attached. In addition, the test strip 12 having a thin plate shape is discharged from the component measurement device 14 at an initial speed to some extent, so that it can be reliably put into a disposal box. Furthermore, when the test strip 12 is inserted, the test strip 12 can be inserted at a correct position without being bent or warped, which is also preferable for measurement.

The component measurement system 10 and the component measurement device 14 described above have the following effects.

A component measurement system 10 of the present embodiment includes: a test strip 12 having a main body portion 16 with a thin plate shape, a flow path 20 that is formed inside the main body portion 16 and extends from a first end portion 12a toward an opposite second end portion 12b in a longitudinal direction of the main body portion 16 and stores a liquid, and an introduction port 18 provided at the first end portion 12a; and a component measurement device 14 that stores the test strip 12 and detects a component in the liquid. The component measurement device 14 of the component measurement system 10 includes: a housing 44 having a distal portion 14a and a proximal portion 14b; an insertion hole 48 that is provided inside the housing 44 and accommodates the test strip 12; a measurement unit that detects a target component in a liquid in at least a part of the flow path 20 in a state in which the test strip 12 is completely accommodated in the insertion hole 48; and a positioning mechanism 34 that maintains an insertion position of the test strip 12. The positioning mechanism 34 includes a gripping mechanism 50 that is provided between the insertion hole 48 and the proximal portion of the housing 44 and grips the test strip 12 while biasing the test strip 12 from the width direction.

In the above component measurement system 10, the direction in which the gripping mechanism 50 biases the test strip 12 is a direction orthogonal to the transmission direction of the measurement light and is a width direction of the test strip 12. Therefore, even when the test strip 12 is gripped by the gripping mechanism 50, the flow path 20 is not deformed in the thickness direction, so that the length of the optical path through which the measurement light is transmitted is not changed. As a result, the component measurement system 10 can accurately measure a concentration of a target component.

In the above component measurement system 10, the gripping mechanism 50 includes a pair of opposing leaf springs 56. The component measurement system 10 can realize the positioning mechanism 34 with a simple device configuration.

In the component measurement system 10 described above, the test strip 12 has an engagement portion 36 that abuts the gripping mechanism 50. The gripping mechanism 50 abuts on the first inclined portion 40a of the engagement portion 36 to bias the test strip 12 toward the proximal portion of the housing 44. The first inclined portion 40a is inclined with respect to the longitudinal center axis 12e of the test strip 12 in a direction of expanding outward in the width direction of the test strip 12 toward the second end portion 12b. The component measurement system 10 positions the test strip 12 in a biased state, so that the test strip 12 can be prevented from rattling in the component measurement device 14 during measurement.

In the component measurement system 10 described above, the positioning mechanism 34 has a receiving portion 52 that abuts on the second end portion 12b of the test strip 12. The gripping mechanism 50 causes the test strip 12 to abut on the receiving portion 52, so that the test strip 12 is positioned in the longitudinal direction. This component measurement system 10 allows more accurate positioning of the test strip 12.

In the component measurement system 10 described above, the component measurement device 14 has a discharge mechanism 54 that pushes out the test strip 12 toward the distal portion of the housing 44. The component measurement system 10 can smoothly discharge the test strip 12 without contacting it after use.

In the component measurement system 10 described above, the engagement portion 36 of the test strip 12 has a second inclined portion 40b between the first inclined portion 40a and the second end portion 12b, with the width of the second inclined portion 40b decreasing toward the second end portion 12b. The gripping mechanism 50 biases the test strip inward in the width direction while abutting on the second inclined portion 40b, thereby biasing the test strip 12 toward the first end portion 12a with respect to the component measurement device 14. This component measurement system 10 can smoothly discharge the test strip 12.

In the component measurement system 10 described above, the second inclined portion 40b of the test strip 12 is inclined by 30° or more with respect to the longitudinal center axis 12e of the test strip 12. The component measurement system 10 can reliably discharge the test strip 12 even if the adhesive is exposed to the side portions 12c and 12d of the test strip 12. That is, even if the side portions 12c and 12d of the test strip 12 are processed as normal cut sections, the test strip 12 can be reliably discharged from the component measurement device 14. As a result, it is not necessary to perform special processing on the side portions 12c and 12d of the test strip 12.

In the component measurement system 10 described above, the component measurement device 14 includes the stopper 48a that restricts a displacement range inward in the width direction of the pair of opposing leaf springs 56 of the gripping mechanism 50, and the tips of the pair of opposing leaf springs 56 are held on both sides in the width direction of the stopper 48a in a state in which the test strip 12 is not inserted. The component measurement system 10 can limit a displacement range of the leaf spring 56 while increasing the biasing force F of the leaf spring 56.

In the component measurement system 10 described above, the test strip 12 has the second inclined portion 40b separated from the second end portion 12b. A small width portion 38 formed with a constant width is provided between the second inclined portion 40b and the second end portion 12b. The side portion of the small width portion 38 is disposed in a range not abutting on the gripping mechanism 50. The component measurement system 10 can prevent the test strip 12 from decelerating due to the contact between the small width portion 38 and the leaf spring 56, and can smoothly discharge the test strip 12.

A component measurement device 14 of the present embodiment is a component measurement device 14 used in a component measurement system 10 that detects a component in a liquid using a test strip 12 including: a main body portion 16 having a thin plate shape; a flow path 20 that is formed inside the main body portion 16, extends from a first end portion 12a toward an opposite second end portion 12b in a longitudinal direction of the main body portion 16, and stores the liquid; and an introduction port 18 provided at the first end portion 12a. The component measurement device 14 includes: a housing 44 having a distal portion 14a and a proximal portion 14b; an insertion hole 48 that is provided inside the housing 44 and accommodates the test strip 12; a measurement unit that detects a component in a liquid in at least a part of the flow path 20 in a state in which the test strip 12 is completely accommodated in the insertion hole 48; and a positioning mechanism 34 that maintains an insertion position of the test strip 12. The positioning mechanism 34 includes a gripping mechanism 50 that is provided between the insertion hole 48 and the proximal portion 14b of the housing 44 and grips the test strip 12 while biasing the test strip 12 in the width direction. The component measurement device 14 can accurately detect a component in a liquid.

Note that the present invention is not limited to the above-described embodiments, and various configurations can be adopted without departing from the gist of the present invention.

## Claims

1. A component measurement system comprising:
a test strip including a main body portion with a thin plate shape, a flow path that is formed inside the main body portion, extends from a first end portion toward an opposite second end portion in a longitudinal direction of the main body portion, and accommodates a liquid, and an introduction port provided at the first end portion; and
a component measurement device that accommodates the test strip and detects a component in the liquid, wherein
the component measurement device comprises:
a housing having a distal portion and a proximal portion;
an insertion hole that is provided inside the housing and accommodates the test strip;
a measurement unit that detects a target component in the liquid in at least a part of the flow path in a state in which the test strip is completely accommodated in the insertion hole; and
a positioning mechanism that maintains an insertion position of the test strip, and
the positioning mechanism includes a gripping mechanism that is provided between the insertion hole and the proximal portion of the housing and grips the test strip while biasing the test strip from a width direction.

2. The component measurement system according to claim 1, wherein the gripping mechanism includes a pair of opposing leaf springs.

3. The component measurement system according to claim 2, wherein the test strip includes an engagement portion that abuts on the gripping mechanism, the gripping mechanism abuts on a first inclined portion of the engagement portion to bias the test strip toward the proximal portion of the housing, and the first inclined portion is inclined with respect to a longitudinal center axis of the test strip in a direction of expanding outward in the width direction of the test strip toward the second end portion.

4. The component measurement system according to claim 3, wherein the positioning mechanism includes a receiving portion that abuts on the second end portion of the test strip, and the gripping mechanism causes the test strip to abut on the receiving portion, so that the test strip is positioned in the longitudinal direction.

5. The component measurement system according to claim 3 or 4, wherein the component measurement device includes a discharge mechanism that pushes out the test strip toward the distal portion of the housing.

6. The component measurement system according to claim 5, wherein the engagement portion of the test strip includes a second inclined portion between the first inclined portion and the second end portion, with a width of the second inclined portion decreasing toward the second end portion, and the gripping mechanism biases the test strip inward in the width direction while abutting on the second inclined portion, to bias the test strip toward the first end portion with respect to the component measurement device.

7. The component measurement system according to claim 6, wherein the second inclined portion of the test strip is inclined by 30° or more with respect to the longitudinal center axis of the test strip.

8. The component measurement system according to claim 7, wherein the component measurement device includes a stopper that restricts a displacement range inward in the width direction of the pair of opposing leaf springs of the gripping mechanism, and tips of the pair of opposing leaf springs are held on both sides in the width direction of the stopper in a state in which the test strip 12 is not inserted.

9. The component measurement system according to claim 8, wherein the test strip includes a small width portion in which the second inclined portion is separated from the second end portion and formed to have a constant width between the second inclined portion and the second end portion, and a side portion of the small width portion is disposed in a range not abutting on the gripping mechanism.

10. A component measurement device for use in a component measurement system that detects a component in a liquid using a test strip including a main body portion with a thin plate shape, a flow path that is formed inside the main body portion, extends from a first end portion toward an opposite second end portion in a longitudinal direction of the main body portion, and accommodates the liquid, and an introduction port provided at the first end portion, the component measurement device comprising:
a housing having a distal portion and a proximal portion;
an insertion hole that is provided inside the housing and accommodates the test strip;
a measurement unit that detects a component in the liquid in at least a part of the flow path in a state in which the test strip is completely accommodated in the insertion hole; and
a positioning mechanism that maintains an insertion position of the test strip, wherein
the positioning mechanism includes a gripping mechanism that is provided between the insertion hole and the proximal portion of the housing and grips the test strip while biasing the test strip in a width direction.
